# EUROPEAN PATENT SPECIFICATION

(11) **EP 3 821 923 B1**
(45) Date of publication and mention of the grant of the patent: **23.07.2025**
(21) Application number: 20216410.9
(22) Date of filing: 26.06.2009
(51) Int. Cl.: A61M 5/142, A61M 5/158

(54) **A CANNULA ASSEMBLY AND A DISPOSABLE INFUSION DEVICE WITH SUCH CANNULA ASSEMBLY**
EINE KANÜLENANORDNUNG UND EINE WEGWERFBARE INFUSIONSVORRICHTUNG MIT EINER SOLCHER KANÜLENANORDNUNG
UN ASSEMBLAGE DE CANULES ET DISPOSITIF JETABLE DE PERFUSION AVEC CET ASSEMBLAGE DE CANULES

(30) Priority: 26.06.2008 US 147306
(43) Date of publication of application: 19.05.2021
(62) Divisional of application: 09771185.7
(73) Proprietor: Ethicon, Inc., Raritan, NJ 08869 (US)
(72) Inventor: CROSS, Brett, Seattle, WA 98119-2814 (US); MARSOT, Travis, Mountain View, CA 94043 (US); CARTER, Brett J., Monroe, WA 98272-2724 (US)
(74) Representative: Carpmaels & Ransford LLP

(56) References cited:
- WO-A2-01/89607
- WO-A2-2007/142890
- US-A1- 2005 101 932
- US-A1- 2008 051 730

## Description

### BACKGROUND OF THE INVENTION

The present invention relates to a cannula assembly for use in infusion devices and more particularly for such devices that enable liquid medicaments to be conveniently and safely self-administered by a patient.

Administration of insulin has traditionally been accomplished using a syringe. Recently, needle carrying pen-like devices have also been employed for this purpose. Both forms of insulin administration require the patients to stick themselves each time they inject insulin, often many times a day. Thus, these traditional forms of insulin administration have been a rather pervasive intrusion in the lives and routines of the patient's who have had to adopt and employ them.

More recently, insulin pumps attached by tubing to an infusion set mounted on the patient's skin have been developed as an alternative form of insulin administration. Such pumps may be controlled by a programmable remote electronic system employing short range radio communication between a control device and electronics that control the pump. While such devices may involve fewer needle sticks, they are expensive to manufacture. They are also complex to operate and cumbersome and awkward to wear. Further, the cost of such devices can be many times the daily expense of using a traditional injection means such as a syringe or an insulin pen.

Devices of the type mentioned above also require a significant amount of training to control and thus use the devices. Great care in programming the devices is required because the pumps generally carry sufficient insulin to last a few days. Improper programming or general operation of the pumps can result in delivery of an excessive amount insulin which can be very dangerous and even fatal.

Many patients are also reluctant to wear a pump device because they can be socially awkward. The devices are generally quite noticeable and can be as large as a pager. Adding to their awkwardness is their attachment to the outside of the patients clothes and the need for a catheter like tubing set running from the device to an infusion set located on the patient's body. Besides being obvious and perhaps embarrassing, wearing such a device can also be a serious impediment to many activities such as swimming, bathing, athletic activities, and many activities such as sun bathing where portions of the patient's body are necessarily uncovered.

In US2008051730A1, there is described an infusion medium delivery system, device and method for delivering an infusion medium to a patient-user, that includes a needle inserter device and method for inserting a needle and/or cannula into a patient-user to convey the infusion medium to the patient-user. The needle inserter device and method operate to insert a needle and cannula into a patient-user's skin and automatically withdraw the needle from the patient-user, leaving the cannula in place and in fluid flow communication with a reservoir. The delivery device may include a base portion and a durable portion connectable to the base portion, and wherein the base portion can be separated from the durable portion and disposed of after one or more specified number of uses. The base portion supports the reservoir and the needle inserter device, while the durable portion supports a drive device for selectively driving the infusion medium out of the reservoir and into the needle and/or cannula.

In view of the above, a more cost effective and simple device has been proposed whereby an injection system is discreetly attached directly to the skin of the patient. The device may be attached to the patient under the patient's clothing to deliver insulin into the patient by the manual pumping of small doses of insulin out the distal end of a temporarily indwelling cannula that is made a part of the pump device. The cannula may be made a part of the drug delivery device before, during or after the attachment of the drug delivery device to the skin of the patient. The device may be made quite small and, when worn under the clothes, entirely unnoticeable in most social situations. It may still carry sufficient insulin to last a patient several days. It can be colored to blend naturally with the patient's skin color so as not to be noticeable when the patient's skin is exposed. As a result, insulin for several days may be carried by the patient discreetly, and conveniently applied in small dosages after only a single needle stick. For a more complete description of devices of this type, reference may be had to co-pending application Serial Number 11/906,130, filed on September 28, 2007 for DISPOSABLE INFUSION DEVICE WITH DUAL VALVE SYSTEM.

The present invention provides further improvement to the cannula assembly for the devices disclosed in the above referenced application. More particularly, the devices disclosed herein provide for improved patient safety and/or convenience. Examples described hereafter provide, for example, improved sealing of the medicament, more convenient cannula deployment, device misuse prevention, primed and dosage ready indication and fluid path occlusion detection. These and other advantages are addressed herein.

### SUMMARY OF THE INVENTION

In one embodiment, a disposable wearable infusion device comprises a body arranged to be adhered to a patient's skin and receiving a cannula to extend from the infusion device to beneath the patient's skin, a reservoir for holding a liquid medicament to be infused into the patient, and a pump that displaces a volume of the liquid medicament from the reservoir to the cannula for delivery to the patient. The body has a port aligned with the cannula and enabling the cannula to assume a deployed position to extend from the infusion device to beneath the patient's skin, and a cover that blocks the port to preclude direct access to the cannula through the port after deployment.

The cover is arranged to block the port subsequent to and as a response to deployment of the cannula. The cannula may be arranged to be deployed with a drive needle that extends through the port and carries the cannula into the deployed position.

The cover may be arranged to block the port upon withdrawal of the needle from the port after deployment of the cannula. The port cover may more particularly be arranged to be held away from the port by the needle prior to cannula deployment. The port cover may be formed of resilient material and arranged to spring over and block the port responsive to the drive needle being withdrawn from the port.

The device body includes a cavity. The device further includes a cannula carrier including the port. The cannula carrier may be arranged to be received within the device cavity when the cannula is deployed. The cannula carrier may include the cover.

The cannula may be arranged to be deployed with a drive needle that carries the cannula carrier and the cannula into the deployed position. The cover may then be arranged to block the port upon withdrawal of the needle from the cannula carrier after deployment of the cannula.

The port cover is preferably arranged to be displaced from the port by the needle prior to cannula deployment. The port cover may be formed of resilient material and arranged to spring over and block the port responsive to the drive needle being withdrawn from the port.

In another embodiment, a disposable wearable infusion device comprises a body arranged to be adhered to a patient's skin. The body has a cavity. The device further comprises a cannula assembly deployed within the device body. The cannula assembly includes a cannula carrier received within the body cavity and a cannula extending from the carrier to being beneath the patient's skin. The cannula carrier includes a port aligned with the cannula that facilitates deployment of the cannula assembly. The device further comprises a reservoir for holding a liquid medicament to be infused into the patient, a pump that displaces a volume of the liquid medicament from the reservoir to the cannula for delivery to the patient, and a port cover that blocks the port to preclude direct access to the cannula through the port after deployment.

The cannula carrier may include the port cover. The cannula may be arranged to be deployed with a drive needle that extends through the port and that carries the cannula carrier and the cannula into the deployed position. The cover may be arranged to block the port upon withdrawal of the needle from the cannula carrier after deployment of the cannula. The port cover is arranged to be displaced from the port by the needle prior to cannula deployment. The port cover is preferably formed of resilient material and arranged to spring over and block the port responsive to the drive needle being withdrawn from the port.

According to the invention as defined by the appended claims, a cannula assembly for use within a disposable infusion device comprises a cannula carrier received within the body cavity, a cannula extending from carrier for being beneath the patient's skin, wherein the cannula carrier includes a port aligned with the cannula that facilitates deployment of the cannula assembly within the device, and a port cover that blocks the port to preclude direct access to the cannula through the port after deployment.

The cannula assembly may be arranged to be deployed with a drive needle that extends through the port and carries the cannula carrier and the cannula into the deployed position within the infusion device. The cover may be arranged to block the port upon withdrawal of the needle from the cannula carrier after deployment of the cannula assembly. The port cover may be arranged to be displaced from the port by the needle prior to cannula deployment. The port cover is preferably formed of resilient material and arranged to spring over and block the port responsive to the drive needle being withdrawn from the port.

### BRIEF DESCRIPTION OF THE DRAWINGS

The features of the present invention are set forth in the appended claims and may best be understood by making reference to the following description taken in conjunction with the accompanying drawings, in the several figures of which like reference numerals identify identical elements, and wherein:
**FIG. 1** is a perspective view of an infusion device embodying the present invention shown without a deployed cannula;
**FIG. 2** is another perspective view of the infusion device of **FIG. 1** shown with a deployed cannula;
**FIG. 3** is an exploded perspective view of the device of **FIG. 1****;**
**FIG. 4** is a sectional view, in perspective, to an enlarged scale, taken along lines 4-4 of **FIG. 1****,** showing the actuation linkages of the device of **FIG. 1** prior to medicament dosage delivery;
**FIG. 5** is another sectional view, in perspective, to an enlarged scale, taken along lines 5-5 of **FIG. 2****,** showing the actuation linkage operation of the device of **FIG. 1** during medicament dosage delivery;
**FIG. 6** is another sectional view similar to that of **FIG. 5****,** in perspective, to an enlarged scale, showing the actuation linkage operation of the device of **FIG. 1** immediately after dosage delivery;
**FIG. 7** is a schematic representation of the valves and pump of the device of **FIG. 1** between medicament dosage deliveries and during the filling of the pump with the medicament;
**FIG. 8** is another schematic representation of the valves and pump of the device of **FIG. 1** during medicament dosage delivery;
**FIG. 9** is a sectional view, in perspective, to an enlarged scale, showing the configuration of the valves of the device of **FIG. 1** during pump filling and prior to medicament dosage delivery;
**FIG. 10** is another sectional view, in perspective, to an enlarged scale, showing the configuration of the valves of the device of **FIG. 1** during dosage delivery;
**FIG. 11** is a top perspective view of the base of the device of **FIG. 1** illustrating various fluid paths within the device;
**FIG. 12** is a partial bottom plan view of the base of the device of **FIG. 1** to illustrate the interior of a prime indicator according to one embodiment thereof;
**FIG. 13** is a bottom plan view of the base of the device of **FIG. 1** illustrating the prime indicator interior covered by a translucent cover according to the above mentioned prime indicator embodiment;
**FIG. 13A** is a bottom view of the device ***110*** illustrating a removable non-adhesive layer overlying an adhesive layer on the device base;
**FIG. 14** is a sectional view in perspective of an occlusion detector and lock-out according to an embodiment of the device of **FIG. 1** shown prior to an intended dosage delivery;
**FIG. 15** is another sectional view in perspective of the occlusion detector and lock-out shown during an intended dosage delivery;
**FIG. 16** is another sectional view in perspective of the occlusion detector and lock-out shown during occlusion detection and just prior to lock-out;
**FIG. 17** is a perspective view of the device of **FIG. 1** and a cannula placement assembly attached thereto ready to provide the device with a cannula according to a still another embodiment;
**FIG. 18** is a sectional view, in perspective, to an enlarged scale, of the device and cannula placement assembly before the cannula is deployed;
**FIG. 19** is a sectional view like that of **FIG. 18****,** showing the device and cannula placement assembly during cannula deployment;
**FIG. 20** is a sectional view like that of **FIG. 18****,** showing the device and cannula placement assembly after cannula deployment;
**FIG. 21** is a sectional view, in perspective, of the cannula placement assembly of **FIG. 17****,** showing the driver prior to cannula deployment;
**FIG. 22** is a sectional view, in perspective, and to an enlarged scale, of the cannula placement assembly of **FIG. 17****,** showing the driver through a plane perpendicular to the sectional plane of **FIG. 21** and prior to cannula deployment;
**FIG. 23** is another sectional view similar to **FIG. 22** showing the cannula placement assembly in an enabled configuration;
**FIG. 24** is a sectional view, in perspective, and to an enlarged scale, showing the cannula placement assembly being released for deploying a cannula;
**FIG. 25** is a sectional view, similar to that of **FIG. 21****,** showing the cannula placement assembly during cannula deployment;
**FIG. 26** is another sectional view, similar to that of **FIG. 21****,** showing the cannula placement assembly after cannula deployment;
**FIG. 27** is a perspective view of the device and cannula placement assembly after cannula deployment and separation of the cannula driver from the device;
**FIG. 28** is another sectional view of the cannula placement assembly and device to an enlarged scale showing the device and driver including details of a cannula needle port cover during cannula delivery;
**FIG. 29** is a sectional view similar to that of **FIG. 28** showing the device and the cannula placement assembly including further details of the cannula needle port cover after cannula deployment;
**FIG. 30** is a sectional view, to an enlarged scale, showing a drive needle assembly including a drive needle and drive needle head according to a further embodiment of the invention; and
**FIG. 31** is a sectional view, to an enlarged scale, illustrating an alternative embodiment of a cannula port cover after cannula deployment.

### DETAILED DESCRIPTION OF THE INVENTION

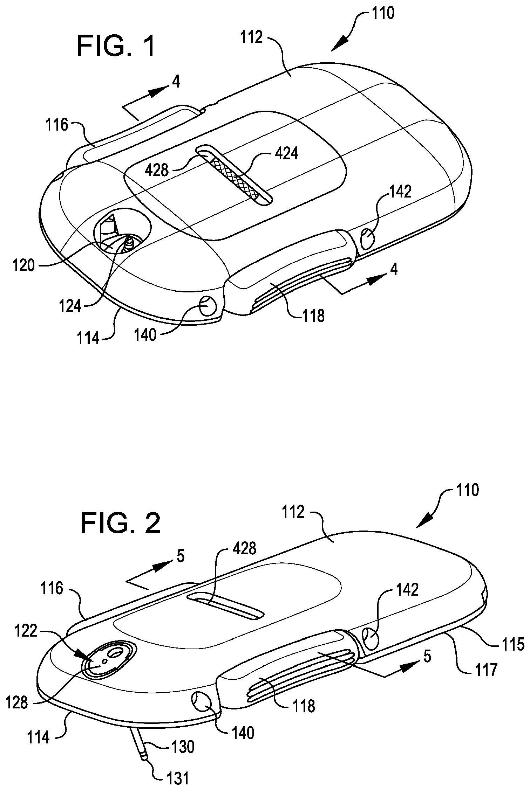

Referring now to **FIGS. 1 and 2****,** they are perspective views of an infusion device ***110*** embodying various aspects of the present invention. **FIG. 1** shows the device prior to receiving and thus without a cannula while **FIG. 2** illustrates the device after having received a cannula ***130*** that has a distal end ***131.*** As may be seen in both **FIGS. 1 and 2****,** the device ***110*** generally includes an enclosure ***112,*** a base ***114,*** a first actuator control button ***116,*** and a second actuator control button ***118.***

The enclosure ***112,*** as will be seen subsequently, is formed by virtue of multiple device layers being brought together. Each layer defines various components of the device such as, for example, a reservoir, fluid conduits, pump chambers, and valve chambers, for example. This form of device construction results in a compact design and enables manufacturing economy to an extent that the device is disposable after use.

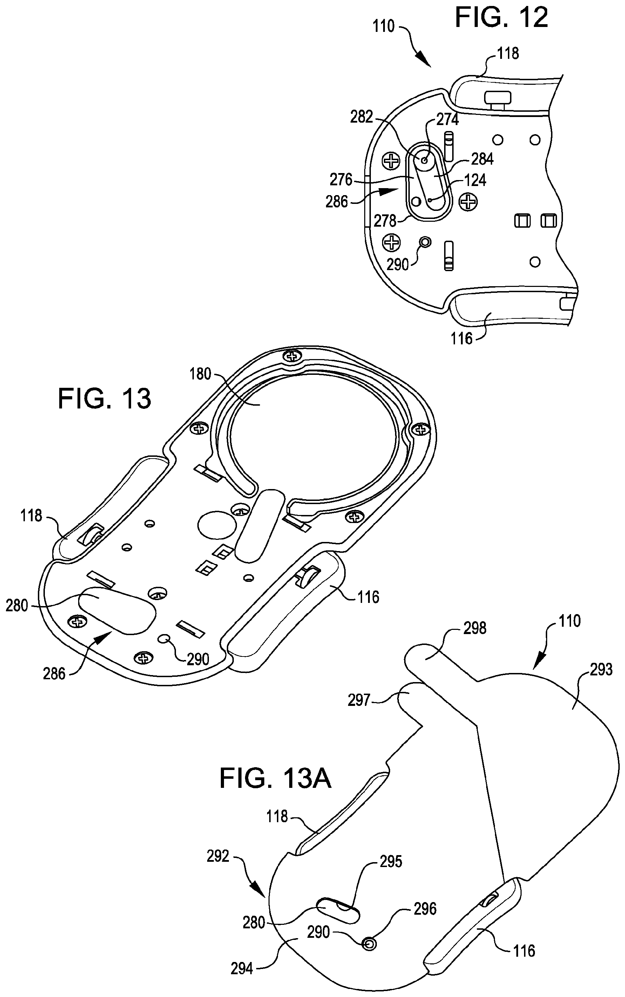

The base ***114*** preferably includes a pad ***115*** attached to the base ***114.*** The pad ***115*** has an adhesive coating ***117*** on the side thereof opposite the base ***114*** to permit the device to be adhered to a patient's skin. The adhesive coating may originally be covered with a releasable cover **292 (****FIG. 13A****)** that may be pealed off of the adhesive layer ***117*** when the patient endeavors to adhere the device ***110*** to their skin.

The device ***110,*** as will be seen herein after is first adhered to the patient's skin followed by the deployment of the cannula ***130*** thereafter. However, it is contemplated herein that various aspects of the present invention may be realized within a device that may alternatively be mated with a previously deployed cannula assembly.

The actuator buttons ***116*** and ***118*** are placed on opposites sides of the device ***110*** and directly across from each other. This renders more convenient the concurrent depression of the buttons when the patient wishes to receive a dose of the liquid medicament contained within the device ***110.*** This arrangement also imposes substantially equal and opposite forces on the device during dosage delivery to prevent the device from being displaced and possibly stripped from the patient. As will be further seen hereinafter, the concurrent depression of the buttons is used to particular advantage. More specifically, the actuator button ***116*** may serve as a valve control which, when in a first position as shown, establishes a first fluid path between the device reservoir and the device pump to support pump filling, and then, when in a second or depressed position, establishes a second fluid path between the device pump and the device outlet or distal end of the cannula to permit dosage delivery to the patient. As will be further seen, a linkage between the control actuator buttons ***116*** and ***118*** permits actuation of the device pump with the actuator control button ***118*** only when the second fluid path has been established by the first actuator control button ***116.*** Hence, the first actuator control button ***116*** may be considered a safety control.

The actuator buttons ***116*** and ***118*** are preferably arranged to require a complete through of their travel to achieve activation of the device pump and thus dosage delivery. This, together with the sudden release of resistance to actuator advancement creates a snap action that provides an advantage in positively knowing that dosage delivery has occurred and that no less than a full dose has been delivered. For more description regarding this feature, reference may be had to co-pending application Serial Number 11/906,102, titled DISPOSABLE INFUSION DEVICE WITH SNAP ACTION ACTUATION, which application is owned by the assignee of this application.

As may be noted in **FIG. 1****,** the device ***110*** includes a cavity ***120*** that is arranged to receive a cannula assembly ***122* (****FIG. 2****)** from which the cannula ***130*** extends. When the cannula is deployed, the outlet ***124*** of the device ***110*** is placed in fluid communication with the cannula ***130*** by a cannula carrier ***128*** of the cannula assembly ***122*** that carries the cannula. When thus deployed, the cannula ***130*** extends from the base ***114*** of the device ***110*** to beneath the skin of the user.

As may further be noted in **FIGS. 1 and 2****,** the enclosure ***112*** of the device ***110*** includes a pair of pockets ***140*** and ***142*** on opposite sides of the second actuator button ***118.*** A similar pair of pockets, not seen in the figure, are also provided on opposite sides of the first actuator button ***116.*** These pockets are used to receive corresponding projections of a cannula placement assembly for releasably joining the cannula placement assembly to the device ***110*** to support cannula deployment as will be described subsequently. As will also be seen, upon cannula deployment, the cannula placement assembly is automatically released from the device by the driver projections being forced from the pockets.

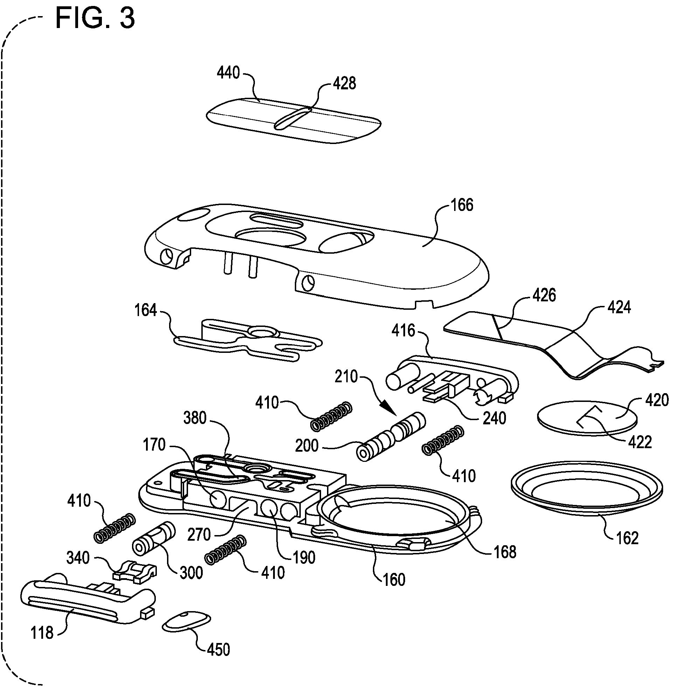

Referring now to **FIG. 3****,** it is an exploded perspective view of the device ***110*** of **FIG. 1****.** It shows the various component parts of the device. The main component parts include the aforementioned device layers including the base layer ***160,*** a reservoir membrane ***162*,** an intermediate layer ***164*** and a top body layer ***166.*** As may also be seen in **FIG. 3****,** the base layer ***160*** is a substantially rigid unitary structure that defines a first reservoir portion ***168,*** a pump chamber ***170,*** and a valve chamber ***190*** that receives a shuttle bar ***200*** of a shuttle valve ***210.*** A reservoir membrane layer ***162*** is received over the reservoir portion ***168*** to form an expandable/deflatable reservoir of the device ***110.*** The base layer ***160*** may be formed of plastic, for example. The base and the top body layer may be joined together, trapping the intermediate layer there between by any means such as with screws, ultrasonic welding or laser welding.

The valve chamber ***190*** is arranged to receive a valve shuttle bar ***200*** carried by and extending from the first actuator button ***116.*** A series of O-rings, to be described subsequently, are seated on the shuttle bar ***200*** to form first, second, and third valves. The actuator button ***116*** also carries a first linkage portion ***240*** of the linkage that permits actuation of the device pump with the actuator control button ***118*** only when the second fluid path has been established by the first actuator control button ***116.*** The first linkage portion ***240*** is received within a suitably configured bore ***270*** formed in the base layer ***160*** and will be described subsequently.

The pump actuator button ***118*** is arranged to be linked to a pump piston ***300*** and a second linkage portion ***340*** to interact with the first linkage portion ***240.*** The pump piston ***300*** is arranged to be received within the pump chamber ***170*** and the second linkage portion ***340*** is arranged to be received within the bore ***270*** for interacting with the first linkage portion ***240.*** O-rings are seated on the piston ***300*** to provide a seal against leakage and to prevent external contaminants from entering the piston chamber.

The intermediate layer ***164*** may be a generally resilient member and received on the base layer ***160*** to cover channels scribed in the base layer as a type of gasket to form fluid channels ***380*** that serve to conduct the medicament from the reservoir to the device outlet and to the distal end ***131* (****FIG. 2****)** of the cannula ***130.*** Springs ***410*** are arranged to spring load the actuator buttons ***116*** and ***118*** away from each other.

The reservoir membrane ***162*** is formed of flexible membrane material and is received over the reservoir portion ***168*** to form the reservoir of the device ***110.*** A rigid plate ***420*** is arranged to be adhered to the reservoir membrane ***162*** of the reservoir. Because the membrane ***162*** is flexible, it will move as the reservoir is filled and emptied. The rigid plate ***420*** will then move with it. The plate ***420*** includes an eyelet ***422*** dimensioned to receive an elongated web ***424*** that forms a part of a medicament level indicator. The web ***424*** carries an indicator line or feature ***426*** that may be read through a window ***428*** of the device top most panel ***440.***

Another component of the device ***110*** is a translucent window ***450*** that is received on the underside of the base ***160.*** As will be seen hereinafter, the window forms a part of a prime indicator. It is formed of a transparent material such as glass or transparent plastic and has a roughened surface rendering it translucent. However, when it is covered with or at least wetted by liquid medicament, it is rendered essentially transparent creating a visually obvious condition and, for example, permitting indicia to be seen beneath it indicating that the conduit to the device outlet is primed and ready to deliver fixed doses of medicament when desired.

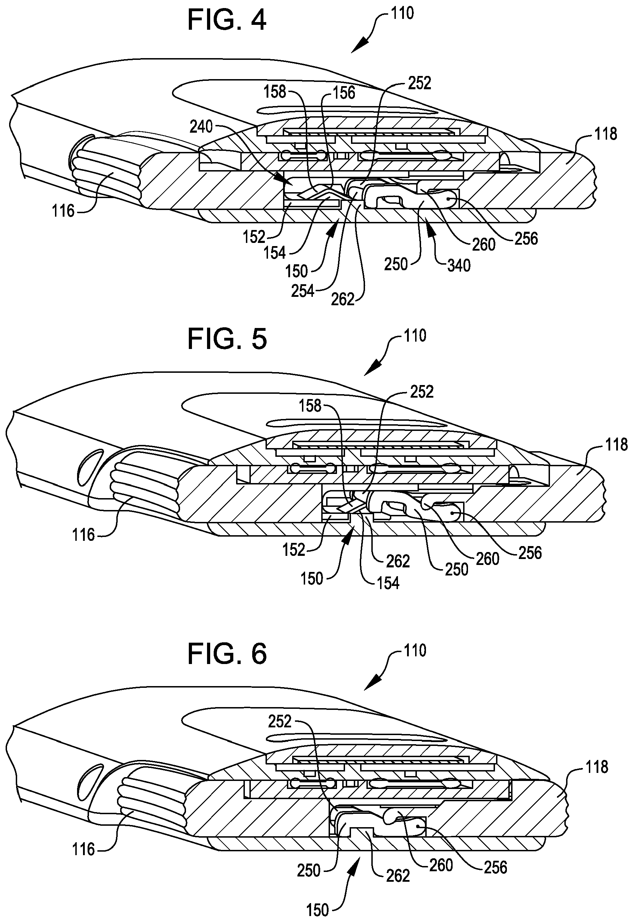

**FIGS. 4-6** show details of the operation of the linkage that permits actuation of the device pump with the actuator control button ***118*** only when the second fluid path from the reservoir to the outlet has been established by the first actuator control button ***116.*** The linkage has been given the general reference character ***150.***

As may be seen **FIG. 4****,** the first actuator button ***116*** has an extension ***152*** that terminates in a block ***154.*** The block ***154*** has a first ramp surface ***156*** and a second ramp surface ***158.*** When the device ***110*** is actuated, the button ***116*** is concurrently depressed with pump button ***118.*** It and its extension ***152*** and block ***154*** are free to move to the right. As seen in **FIGS. 4 and 5****,** the pump actuator button ***118*** has parallel extensions ***250*** and ***252*** which are joined and separated be a rod member ***254.*** The extensions ***250*** and ***252*** are pivotally mounted to pivot about a pivot point ***256.*** Another extension ***260*** of the pump actuator button ***118*** spring biases the extensions ***250*** and ***252*** as shown in **FIG. 4****.** As seen in **FIG. 4****,** the extensions ***250*** and ***252*** abut an abutment ***262*** which they must clear to enable the actuator ***118*** to be moved to the left. As shown in **FIG. 5****,** as the button ***116*** is depressed, its extension ***152*** moves to the right causing the first ramp surface ***156*** to engage the rod member ***254.*** Continued movement of the button causes the rod member ***254*** to ride up the first ramp surface ***156*** which in turn causes the extensions ***250*** and ***252*** to begin to move slightly to the left and bend upward against the loading of extension ***260.*** Eventually, the rod member ***254*** rides up the length of the first ramp ***156*** and down the second ramp ***158*** causing the extensions ***250*** and ***252*** to clear the abutment ***262*** and continue their travel to the left until the extensions are received on the opposite side of the abutment as shown in **FIG. 6****.** The pump button ***116*** has now been fully depressed to deliver a dose of measured medicament. When the ends of extensions ***250*** and ***252*** totally clear the abutment ***262,*** they will snap behind the abutment ***262*** as shown in **FIG**. **6** and become temporarily locked. Meanwhile, the rod member ***254*** has traversed all the way down the second ramp surface ***158.*** The buttons ***116*** and ***118*** are now fully depressed.

Hence, from the above, it may be seen that the pump button ***118*** could not at first move freely while the first actuator button ***116*** which operates the valves could. As a result, the pump actuation lags behind the valve actuation. This enables the device outlet to be sealed from the reservoir and the pump connected to the outlet before the pump is permitted to pump any medicament to the outlet. Hence, the device establishes a medicament delivery flow path to the cannula before the pump is able to begin pumping the medicament to the patient. Thus, it is assured that there is never an open unobstructed pathway between the reservoir and the fluid outlet. Also, by assuring that the pump only draws fluid from the reservoir when the pathway to the outlet is sealed off, it is also assured that a precise amount of fluid is moved with each pump cycle. This operation is completely timed by the linkage just described and occurs quickly, appearing to the patient that both actuator buttons are moving at the same rate.

When the extensions ***250*** and ***252*** of the pump button clear the abutment ***262,*** they become locked in a snap action. This provides positive feedback to the patient that a dosage of medicament was delivered as desired. It also causes a full dose to be delivered. By virtue of the snap action of the pump actuator, only full doses may be administered.

When the medicament has been delivered, the spring loading of the actuator buttons returns the buttons to their first or initial position. During this time, the same timing provided by the block ***154*** is used for recharging the pump. More specifically, ramp ***158*** unlatches the ends of extensions ***250*** and ***252*** by lifting rod member ***254.*** While the extensions ***250*** and ***252*** are being lifted by the ramp ***158,*** the valve control button ***116*** is returning to the left to cause the outlet to be disconnected from the pump before the reservoir is reconnected to the pump for charging, thus sealing the outlet from both the pump and the reservoir before the reservoir is connected to the pump for recharging. This assures that the pump does not pull medicament from the patient but only from the reservoir. As the pump returns, a full dose of the medicament is drawn up into the piston chamber ***170*** to ready the device for the next dosage delivery.

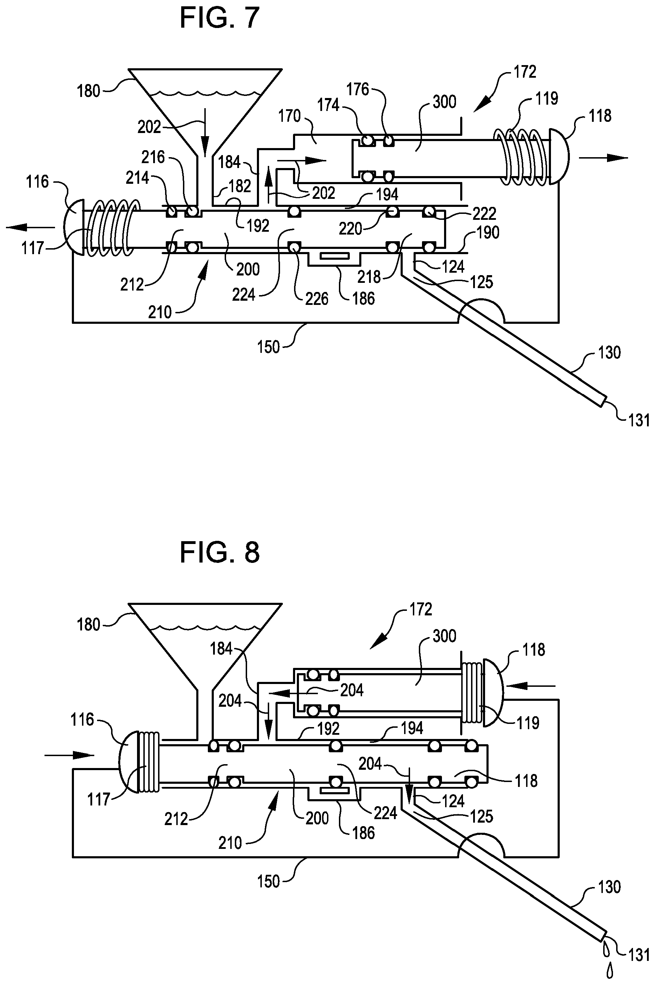

Referring now to **FIGS. 7 and 8****,** they are schematic representations of the valves and pump of the device of **FIG. 1** between medicament dosage filling **(****FIG. 7****)** and medicament dosage delivery **(****FIG. 8****)** As may be seen in **FIGS. 7 and 8****,** the device ***110*** further includes a reservoir ***222,*** a pump ***224,*** and the cannula ***130.*** The reservoir ***222*** may be formed as shown in **FIG. 3** by the combination of the device base ***160*** and the flexible membrane ***162.*** The device further includes the shuttle valve ***210*** including shuttle bar ***200.*** The shuttle bar ***200*** is shown within the valve chamber ***190.*** The shuttle bar *200* and O-rings ***214*** and ***216*** form a first valve ***212,*** shuttle bar ***200*** and O-rings ***220*** and ***222*** form a second valve ***234*** and shuttle bar ***200,*** O-ring ***226*** and a bypass channel ***186*** form a third valve ***224.*** Although O-rings are used herein to form seals, other types of valve construction may employ forms of seals other than O-rings without departing from the invention.

The pump piston ***300*** is within the piston camber 170 to form a piston pump ***172.*** The actuator control button ***218*** is directly coupled to and is an extension of the pump piston ***226.*** It may also be noted that the actuator buttons ***116*** and ***118*** are spring loaded by springs ***117*** and ***119,*** respectively. The springs are provided for returning the actuator buttons to a first or start position after a dosage is administered.

A fluid conduit ***182*** extends between the reservoir *180* and the valve ***212.*** An annular conduit ***192*** extends between the O-rings ***216*** and ***226,*** and an annular conduit ***194*** extends between the O-rings ***226*** and ***220.*** A fluid conduit ***184*** provides a fluid connection between the reservoir ***180*** and the annular conduits ***192*** and ***194*** depending upon the position of the shuttle valve ***210.*** Also illustrated in **FIG. 7** is the linkage ***150*** that assures that the shuttle valve ***210*** is actuated before the piston pump ***172*** is actuated to provide a dose of medicament.

In **FIG. 7****,** the valves are shown in a first configuration immediately after having returned to their first position following a dosage delivery. After the return of the valves, the linkage ***150*** permits the pump actuator ***118*** and piston ***300*** to return for refilling the pump chamber ***300*** in ready for the next medicament dosage delivery. During their return, the medicament flows as indicated by arrows ***202*** from the reservoir ***180,*** through the conduit ***182,*** through the annular channel ***192,*** through conduit ***184,*** and into the pump chamber ***170.***

As may be noted, when in the first position, the valves **218** and **224** isolate the outlet ***124*** from both the reservoir **180** and the piston pump ***118.*** Having two such valves isolate the outlet ***124*** when the valves are in the first configuration provides an added degree of safety from medicament being inadvertently delivered to the patient between dosage deliveries. For example, this provides additional safety that the liquid medicament is not accidentally administered to the patient notwithstanding the inadvertent application of pressure to the reservoir. In applications such as this, it is not uncommon for the reservoir to be formed of flexible material. While this has its advantages, it does present the risk that the reservoir may be accidentally squeezed as it is worn. Because the valves ***118*** and ***124*** isolate the outlet ***124*** when the valves are in their first configuration, this redundant protection assures that pressure, accidentally applied to the reservoir, will not cause the fluid medicament to flow to the cannula.

In addition to the linkage ***150*** preventing return of the piston ***300*** until after the valves return to their first and start positions, the O-rings on the shuttle bar ***200*** are also spaced apart to insure that the valves ***218*** and **224** isolate the outlet ***124*** from the pump ***172*** and reservoir ***180*** before the pump is again connected to the reservoir. The O-ring spacing thus effectively forms a second linkage to assure that the cannula ***130*** is connected to the pump ***172*** only when a dosage is to be delivered and that it is never connected to the reservoir ***180.***

In operation, the pump chamber ***170*** is first filled as the actuator button ***118*** returns to the first position after having just delivered a medicament dosage. In this state, the shuttle valve ***210*** is set so that the first valve ***212*** will be open and the second and third valves ***218*** and ***224*** will be closed. This establishes a first fluid path indicated by arrows ***202*** from the reservoir ***180*** to the pump chamber ***170*** to fill the piston pump ***172.*** When the patient wishes to receive another dose of medicament, the actuator buttons are concurrently pressed. The aforementioned linkages, including linkage ***150,*** cause the first valve ***212*** to close and the second and third valves ***218*** and ***224*** to thereafter open. Meanwhile, actuation of the pump ***172*** is precluded until the first valve ***212*** is closed and the second and third valves ***118*** and ***224*** are opened. At this point a second fluid path indicated by arrows ***204*** is established from the pump chamber ***170*** to the cannula ***130.*** The medicament is then administered to the patient through the distal end ***131*** of cannula ***130.***

Once the medication dosage is administered, the piston ***330,*** and thus the actuator button ***118,*** is returned under the spring pressure of spring ***119*** to its initial position. During the travel of the piston back to its first position, a given volume of the liquid medicament for the next dosage delivery is drawn from the reservoir into the pump chamber ***170*** as described above to ready the device for its next dosage delivery.

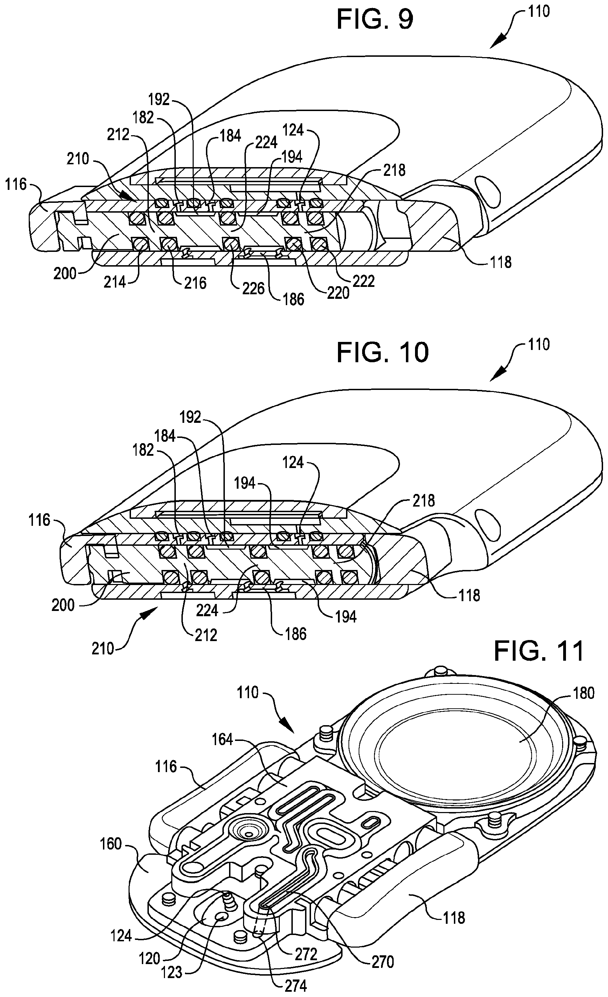

Referring now to **FIG. 9****,** it is a sectional view in perspective showing the valve configuration of the device ***110*** of **FIG. 1** during medicament filling of the pump chamber ***170*** immediately after a dosage delivery. Here, it may be clearly seen that the first actuator button ***116*** is directly coupled to the shuttle bar ***200*** of the valves ***212, 218,*** and ***224.*** Above the valves are the conduits from the reservoir, from the pump, and to the cannula. More particularly, the conduit ***182*** is in fluid communication with the reservoir, the conduit ***184*** is in fluid communication with the pump, and the conduit ***124*** is in fluid communication with the cannula. The valves are shown with the first valve ***212*** opened, communicating reservoir conduit ***182*** with the pump conduit ***184*** through channel ***192,*** the second valve ***218*** closed and blocking the conduit ***124*** to the cannula, and the third valve ***224*** closed and blocking both the reservoir conduit ***182*** and the pump conduit ***184*** from the cannula conduit ***124.*** This permits medicament to flow from the reservoir through conduit ***182,*** through channel ***192,*** and to the pump chamber ***170*** through conduit ***184*** as the actuator button ***116*** returns to its first position. Hence, the pump chamber is filled and ready for the next dosage delivery.

Referring now to **FIG. 10****,** it is a sectional view in perspective similar to that of **FIG. 9** but showing the valve configuration of the device ***110*** of **FIG. 1** during medicament delivery. Here, the valves are shown with the first valve ***212*** closed and blocking the reservoir conduit ***182,*** the second valve ***218*** opened permitting the outlet conduit ***124*** to communicate with the annular conduit ***194,*** and the third valve ***224*** opened permitting medicament to flow from the annular conduit ***192,*** through bypass ***186,*** and to annular conduit ***194.*** Thus, medicament is permitted to flow from the pump conduit ***184,*** through annular conduit ***192,*** through the bypass ***186,*** through annular conduit ***194,*** and into the outlet conduit ***124*** to administer the fixed volume dosage. As previously mentioned, the O-rings defining the first valve ***212,*** the third valve ***224,*** and the second valve ***218*** are spaced apart so that conduit ***182*** is blocked before conduits ***184*** and ***124*** are connected together through the valves ***224*** and ***218.***

**FIG. 11** is a top perspective view of the base ***160*** of the device ***110*** of **FIG. 1****.** Carried on the base ***160*** is the intermediate layer ***164.*** Together, the base ***160*** and intermediate layer ***164*** define numerous fluid conduits within the device ***110.*** One such fluid conduit is designated with reference character ***270*** in **FIG. 11****.** The conduit ***270*** is within the fluid path that leads to the outlet ***124.*** It is in the downstream portion of that path and takes a bend at ***272*** towards the bottom side of the base ***160*** where it, through an opening ***274,*** enters a chamber ***276* (****FIG. 12****).** The chamber ***276*** communicates with the device outlet ***124*** that projects into the aforementioned cavity ***120.*** When the cannula ***130* (****FIG. 13****)** is placed, it extends through an opening ***123*** in the cavity to beneath the patient's skin and communicates with the outlet ***124*** as described herein after.

The chamber ***276*** is partly defined by a seal rim ***278*** which receives a translucent cover ***280* (****FIG. 13****).** The top wall of the chamber ***276*** has an inverted cone shaped surface ***282*** portion and a tapered portion ***284*** to the outlet ***124.***

The translucent cover may be formed of transparent plastic wherein the surface that faces the chamber is roughed in a manner that renders the plastic cover translucent. The upper surface of the chamber is preferably coated with indicia which may, for example, be a color, such as blue. When the chamber is empty, the blue indicia will not be readily seen because the rough surface of the cover has rendered the cover translucent. However, when the chamber ***276*** is filled with a liquid, such as the liquid medicament, the cover ***280*** will become more transparent allowing the blue indicia to be readily seen. The chamber ***276*** and cover ***280*** thus form a prime indicator ***286*** adjacent the outlet ***124.*** More particularly, the prime indicator ***286*** is immediately adjacent the outlet ***124*** since when the chamber is filled and the indicia readily seen, it will be known that the conduits and cannula are sufficiently prime with medicament to permit a full dosage to be delivered upon the next activation of the device ***110.***

In use, it is contemplated that the reservoir be filled through a fill port ***290*** on the bottom of the device ***110*** before the device is deployed on the patient's skin. After the device is filled, the translucent cover or window ***280*** may be viewed during device priming. During the priming process, the actuators ***116*** and ***118*** may be depressed a number of times until the blue indicia on the top surface portions ***282*** and ***284*** of the chamber are seen through the window ***280.*** This provides an indication to the user that the chamber ***276,*** and more importantly, the conduits are sufficiently primed and full with medicament to enable actual dosage delivery upon the next actuation of the device ***110.*** Hence, a prime indicator ***286*** is provided immediately adjacent the outlet ***124.***

In **FIG. 13A****,** it may be seen that the removable non-adhesive layer ***292,*** in accordance with this embodiment, includes two portions, a first portion ***293*** and a second portion ***294.***Each of the first and second layer portions ***293*** and ***294,*** respectively, includes a tab ***298*** and ***297*** respectively that extend beyond the margins of the device base. This permits the tabs ***297*** and ***298*** to be grasped for effortless removal of the removable non-adhesive layer portion ***294*** and ***293.*** The layer portion ***293*** includes cutouts. The cutouts ***295*** and ***296*** extend through the pad ***115* (****FIG. 2****)** to provide access to the device fill port ***290*** and the prime indicator window ***280.*** Hence, as described above, the device ***110*** may be filled and primed for use before the removable layer portions ***293*** and **294** are removed for adhering the device to a patient's skin.

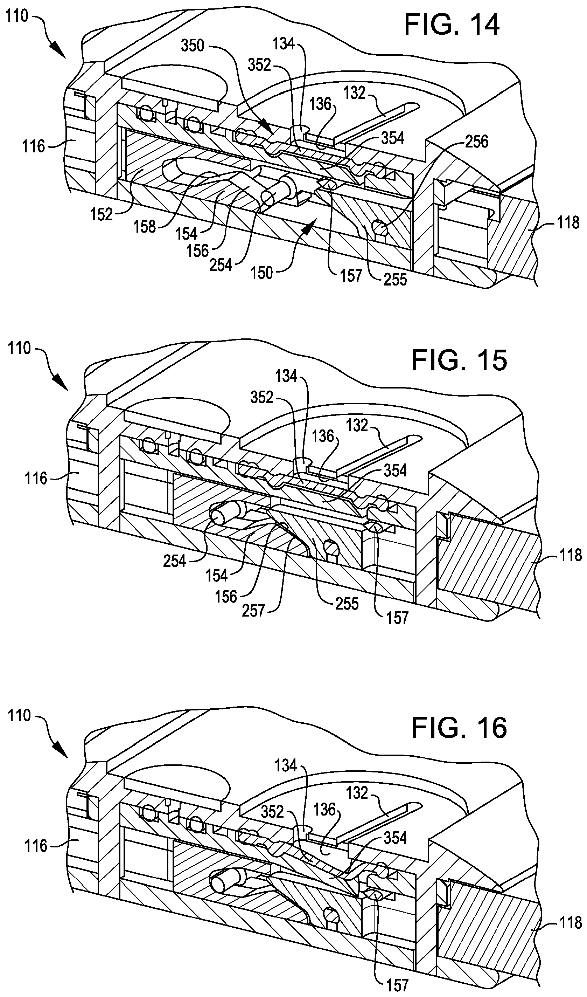

**FIGS. 14-16** are sectional views in perspective of an occlusion detector and lock-out ***350*** according to an embodiment of the device of **FIG. 1****.** **FIG. 14** shows the occlusion detector and lock-out ***350*** prior to an intended dosage delivery, **FIG. 15** shows the occlusion detector and lock-out ***350*** during to an intended dosage delivery, and **FIG. 16** shows the occlusion detector and lock-out ***350*** during occlusion detection and just prior to lock-out.

The occlusion detector and lock-out ***350*** serves to detect, during each dosage delivery, blockages between the shuttle valve and the distal end ***131*** of the cannula ***130* (****FIG. 8****).** Such blockages are most likely to occur within the cannula and may result in an improper amount of medicament being delivered during an attempted dosage delivery. For example, the user might have an erroneous belief that a full dosage had been delivered when, in fact, a dosage of insufficient quantity had been delivered. As will be seen subsequently, the occlusion detector is responsive to pressure within the pathway from the shuttle valve to the outlet and cannula that occurs during an attempted dosage delivery when a blockage exists.

The occlusion detector ***350*** has an inlet ***132*** that communicates with the outlet ***124* (****FIG. 10****)** of the shuttle valve ***210*** and an outlet ***134*** that communicates with the prime indicator ***286* (****FIG. 12****).** Between the inlet ***132*** and the outlet ***134*** is a channel ***136.*** Hence, the channel ***136*** is within the fluid path from the valve to the outlet ***125*** of the device that may be coupled to the cannula ***130* (****FIGS. 7 and 8****).** The occlusion detector ***350*** also includes a resilient diaphragm ***352*** over which the channel ***136*** extends. Adjacent the diaphragm ***352*** is a deflectable stop ***354.*** As will be seen subsequently, when there is an occlusion within the fluid path, an attempted dosage delivery results in an increased back pressure against the diaphragm ***352*** and the deflectable stop ***354.*** This causes the deflectable stop ***354*** to engage a projection ***157*** of the linkage 150. This disables the linkage ***150*** that controls the interaction of the actuator buttons ***116*** and ***118*** and that causes the actuator buttons ***116*** and ***118*** to become locked.

More particularly, as may be further seen in **FIG. 14****,** the linkage ***150*** is adjacent the occlusion detector ***350.*** When the actuator buttons are depressed, the projection ***157,*** carried by the extension ***152*** of the valve actuator button ***116,*** slides across block ***255*** carried on pivot ***256.***

As may be seen in **FIG. 15****,** upon further depression of buttons ***116*** and ***118,*** the projection ***157*** slides past the distal end of the deflectable stop ***354.*** It may also be seen that, as previously described, the rod member ***254*** has ridden up the first ramp ***156*** and down the second ramp ***158*** of block ***154.*** This causes the first ramp *156* to engage a ramp surface ***257*** of block ***255.***

If no occlusion exists within the fluid path to the cannula, the blocks ***154*** and ***255*** and the actuator buttons ***116*** and ***118*** will return as previously described under spring pressure. However, if an occlusion does exist within the fluid path to the cannula, back pressure will quickly build in the channel ***136*** to cause the deflectable stop ***354*** to be deflected downwardly. This is shown in **FIG. 16****.** The deflectable stop ***354*** is now aligned with the projection ***157.*** When, under spring pressure, the actuator buttons **116** and ***118*** are urged to their starting positions, the projection ***157*** will be caught by the deflectable stop ***354*** to lock the device and preclude its further use. Still further, the deflectable stop ***354*** or the projection ***157*** may be breakable or permanently deformable upon their engagement. This would render the linkage ***150*** jammed and irreversibly locked to permanently preclude further use of the device.

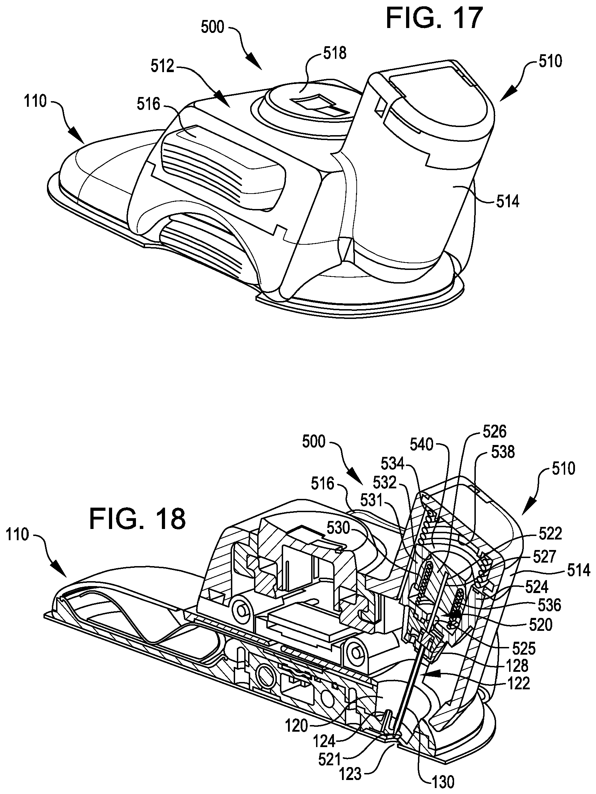

Referring now to **FIG. 17****,** it is a perspective view of the device ***110*** of **FIG. 1** with a cannula placement assembly ***500*** releasably attached thereto ready to provide the device with a cannula according to a still another embodiment. It is contemplated that the device ***110*** and cannula placement assembly ***500*** be pre-attached to each other upon delivery to a patient and that upon placement of the cannula, the cannula placement assembly ***500*** be automatically separated from the device ***110.***

The cannula placement assembly ***500*** generally includes a cannula driver portion ***510*** and an actuation portion ***512.*** The cannula driver portion ***510*** is generally cylindrical in shape including a drive cylinder ***514.*** The actuation portion includes an actuator button ***516*** and a safety control button ***518.*** As will be seen subsequently, the safety control button ***518*** must first be depressed into a locked depressed position before the actuator button ***516*** may be depressed to set the cannula placement sequence into motion.

**FIG. 18** is a sectional view, in perspective, to an enlarged scale, of the device ***110*** and cannula placement assembly ***500*** before the cannula is deployed. As may be noted in **FIG. 18****,** the interior components of the cannula driver portion ***510*** includes a plunger ***524,*** a needle assembly ***520,*** and the cannula assembly ***122.***

The cannula assembly, as previously described, includes the cannula ***130,*** of the type known in the art, and a cannula carrier ***128.*** The cannula carrier ***128*** is arranged to be received within the cavity ***120*** into which the device outlet **124** projects. Once the cannula carrier ***128*** is received into the cavity ***120,*** the cannula carrier ***128*** establishes a fluid path from the outlet ***124*** to the cannula ***130.***

The needle assembly ***520*** includes a drive needle ***522.*** The drive needle ***522*** includes a needle head assembly ***526*** and a needle shaft ***527.*** Before the cannula ***130*** is placed, the cannula assembly ***122*** is carried by the needle assembly ***520*** and more specifically, by the cannula ***130*** being received on the drive needle ***522*** with the cannula carrier ***128*** being immediately adjacent the drive plunger ***524*** as shown.

The needle head assembly ***526*** is initially seated within the drive plunger ***524.*** To that end, the needle head assembly ***526*** is within the plunger ***524*** and has an exterior surface ***530*** that conforms to the interior surface ***532*** of a wall portion ***531*** of the plunger ***524.*** The needle shaft ***527*** extends from the needle head assembly ***526,*** through an opening of the plunger ***524,*** through the cannula carrier ***128,*** and finally through the cannula ***130*** to terminate at a tip ***521.*** During cannula placement, the cannula ***130*** and drive needle ***522*** extend through the opening ***123*** of the device ***110.***

The cannula driver ***510*** further includes a first drive spring ***534*** and a second drive spring ***536.*** The first drive spring is originally compressed between an inner end wall ***538*** of the drive cylinder ***514*** and a top surface ***540*** of the plunger ***524.*** The first drive spring ***534*** serves to drive the plunger ***524*** downwardly to in turn drive the cannula ***130*** and the drive needle ***522*** through the opening ***123.***

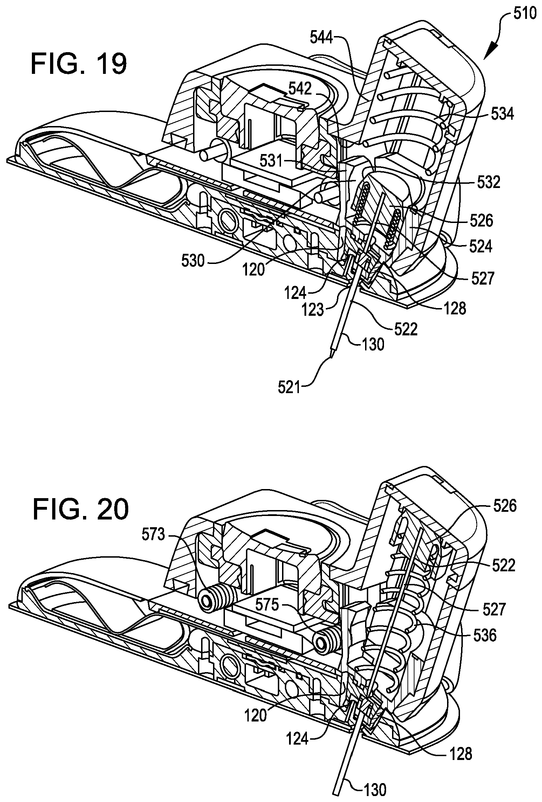

The plunger ***524*** is released by the actuator button ***516*** being depressed. As will be seen subsequently, the actuator button ***516*** includes a portion that interferes with a shoulder of the plunger ***524.*** When the actuator button is depressed, that interference is resolved and the drive carrier ***524*** is free to move downwardly under the influence of the first drive spring ***534.*** The needle tip ***521*** pierces the patient's skin and the lower portion of the needle shaft ***527*** and cannula ***130*** are placed beneath the patient's skin. The cannula ***130*** and needle ***522*** quickly reach the fully driven, temporary, configuration shown in **FIG. 19****.**

In **FIG. 19****,** it may be seen that the cannula ***130*** and needle shaft ***527*** upon which the cannula ***130*** is carried have been driven through the opening ***123*** of the device ***110.*** The first drive spring ***534*** is now in an extended condition.

When the cannula driver ***510*** reaches the configuration shown in **FIG. 19****,** the wall portion ***531*** of the plunger ***524*** clears a shoulder ***542*** of a short wall portion ***544*** of the drive cylinder ***514.*** The wall portion ***531*** springs outwardly separating the conformal surfaces ***530*** and ***532*** of the wall portion ***531*** and the needle head ***526,*** respectively. This frees the needle head ***526*** from the plunger ***524.*** The needle head ***526*** is now free to move upwardly out of and away from the plunger ***524*** under the influence of the second drive spring ***536.***

As the needle head ***526*** moves upwardly, it of course takes the needle shaft ***527*** with it. When the needle head ***526*** reaches the extent of its travel **(****FIG. 20****)** by the extension of the second drive spring ***536*** (the first drive spring ***534*** has been omitted from the figure for clarity), the needle shaft ***527*** is fully extracted from the cannula ***130*** leaving only the cannula ***130*** beneath the patient's skin. The cannula carrier ***128*** has also been received within the cavity ***120*** of the device ***110.*** As the cannula carrier ***128*** is received within the cavity ***120,*** the device outlet ***124*** is placed into fluid communication with the cannula ***130*** through the cannula carrier ***128.***

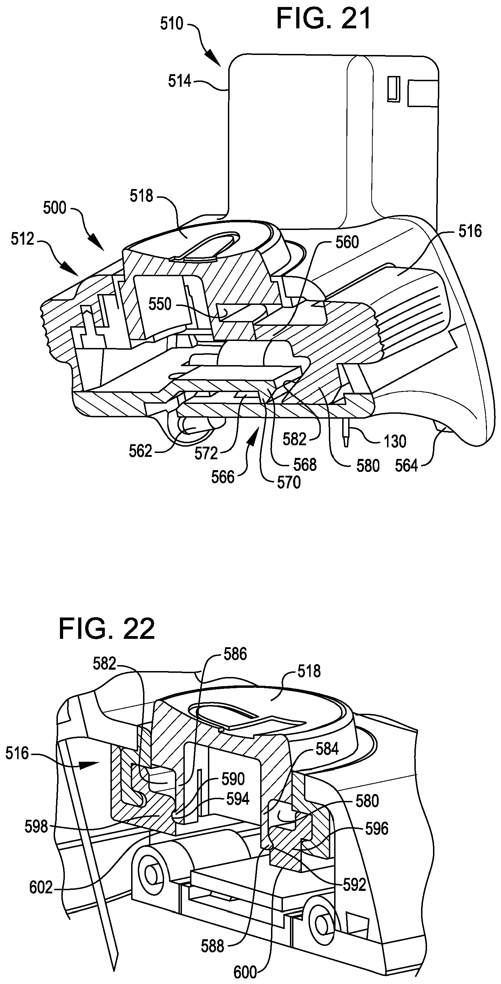

**FIG. 21** is a sectional view, in perspective, showing details of the actuator portion ***512*** of the cannula placement assembly ***500*** of **FIG. 17****,** as configured prior to cannula deployment. As previously described, the actuator portion ***512*** includes the safety control button ***518*** and the actuator ***516.***

The safety control button includes a recess ***550*** and the actuator button ***516*** includes an extension ***560.*** Before actuation, the extension ***560*** of the actuator ***516*** abuts the safety control button to preclude the depressing of the actuator ***516.*** However, when the safety control button is depressed, as will be seen subsequently, the recess ***550*** is brought into alignment with the extension ***560*** to permit the actuator ***516*** to be depressed for setting into motion the placement of the cannula ***130*** as previously described.

As may also be noted in **FIG. 21****,** the cannula placement assembly ***500*** further includes a plurality of projections **562** and ***564.*** It is also contemplated that the cannula placement assembly ***500*** includes two additional such projections. The projections are arranged to be received within corresponding pockets of the wearable infusion device to permit the cannula placement assembly ***500*** to be releasably carried on the device ***110*** as shown, for example, in **FIG. 17****.** For example, projection ***562*** is intended to be received by pocket ***140*** of the device ***110*** of **FIGS. 1 and 2****.** The distance between the projections **562** and ***564*** is maintained by a latch ***566*** that includes a bendable arm ***568*** and a catch ***572.*** The bendable arm includes a hook ***570*** at its distal end that is held by the catch ***572.*** At least one urging member in the form of springs ***573*** and ***575* (****FIG. 20****)** serve to provide a continuous force for separating the projections ***562*** and ***564.*** That force also maintains the hook ***570*** locked onto the catch ***572,*** and the hook ***570*** being locked onto the catch ***572*** maintains the projections within their respective pockets to releasably maintain the cannula placement assembly ***500*** on the device ***110.***

The actuator ***516*** still further includes a latch release projection ***580.*** The latch release projection ***580*** includes a ramped surface ***582*** arranged to engage and bend the bendable arm ***568*** as the actuator ***516*** is depressed. Eventually, sufficient depression of the actuator ***516*** bends the arm ***568*** sufficiently to cause the hook ***570*** to clear the catch ***572.*** The actuator is shaped in such a manner that, as it is depressed in a single motion, it causes release and placement of the cannula before the hook ***570*** clears the catch ***572.*** When the hook ***570*** clears the catch ***572,*** the latch ***566*** is released and the projections, including projections ***562*** and ***564*** are forced apart under spring pressure to cause the cannula placement assembly ***500*** to be separated from the device ***110.***

**FIG. 22** is a sectional view, in perspective, and to an enlarged scale, of the cannula placement assembly of **FIG. 17****,** showing the assembly through a plane perpendicular to the sectional plane of **FIG. 21** and prior to cannula deployment. Here it may be seen that the control button ***518*** includes a pair of chordal recesses ***580*** and ***582*** that defined bendable legs ***584*** and ***586*** respectively. Each of the legs ***584*** and ***586*** includes a projecting foot ***588*** and ***590*** respectively. When the safety control button ***518*** is in a raised initial position as shown in **FIG. 22****,** the foot ***588*** is confined within a slot ***592*** of an extension ***598*** of the actuator button ***516*** and the foot ***590*** is confined within a slot ***594*** of another extension ***598*** of the actuator button ***516.***

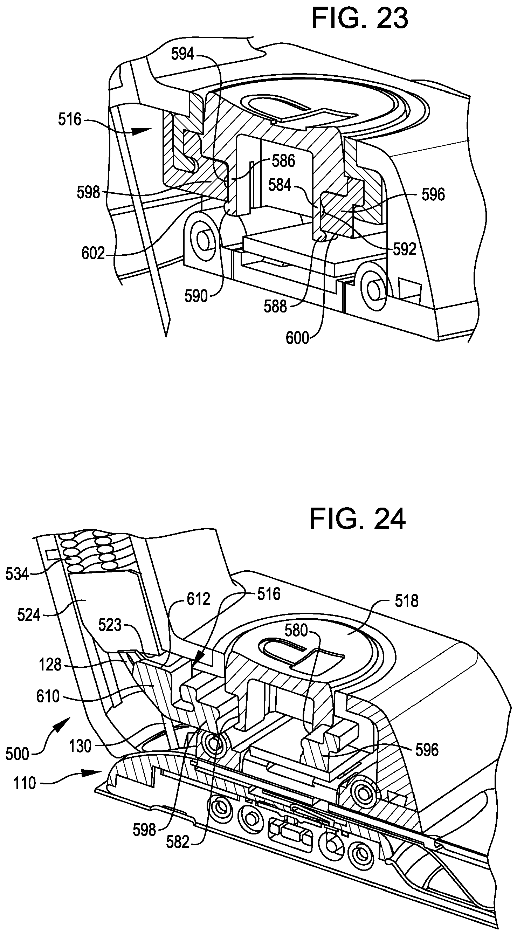

When the control button ***518*** is depressed, the legs ***580*** and ***586*** permit the feet ***588*** and ***590*** to slide out of their respective slots ***592*** and ***594,*** respectively. They then progress downwardly until the legs ***584*** and ***586*** and feet ***588*** and ***590*** wrap about the extensions ***596*** and ***598*** of the actuator ***516.*** The feet ***588*** and ***590*** become confined beneath bottom surfaces ***600*** and ***602,*** respectively, of the actuator button extensions ***596*** and ***598,*** respectively to lock the safety control ***518*** in the enable configuration of **FIG. 23****.**

In **FIG. 23** it may be clearly seen that the feet ***588*** and ***590*** are beneath and confined by the bottom surfaces ***600*** and ***602,*** respectfully, of the actuator button extensions ***596*** and ***598.*** The bendable legs ***584*** and ***586*** cause the control button ***516*** to enter this configuration with a snap action. This provides positive feedback to the user that the control button has been successfully depressed and that the cannula placement assembly is now ready for the depression of the actuator button ***516.*** The confinement by the actuator extensions ***596*** and ***598*** of the feet ***588*** and ***590*** causes the control button ***518*** to be locked in the depressed enable state. This conveys to a user after use that the cannula placement assembly is not to be reused and even assists in making such reuse impossible or at least difficult.

With the control button now depressed and engaged in the enable configuration, the extension ***560*** of the actuator button ***516* (****FIG. 21****)** will now be aligned with the slot ***550*** of the control button ***518.*** This permits the actuator to be depressed and actuated.

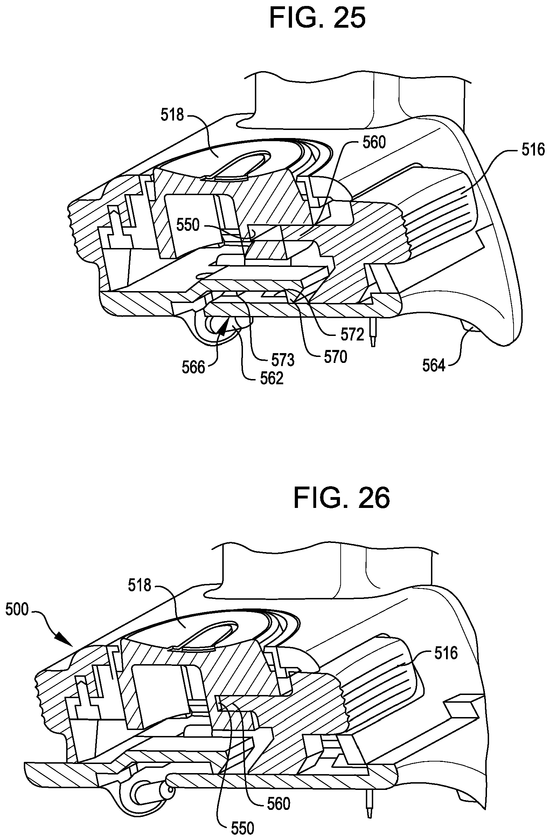

**FIGS. 24** **and** **25** are sectional views showing the cannula placement assembly ***500*** as the actuator button ***516*** is being depressed. In **FIG. 25** it may be seen that the extension ***560*** of the actuator button ***516*** is aligned with and entering into the slot ***550*** of the control button ***518.*** As this occurs, the extension **598** slides in the chordal recess ***582*** of the control button ***518*** and the extension ***596*** slides in the chordal recess ***580*** of the control button.

The extension ***598*** further includes a wing ***610*** having an upper surface ***612*** that normally engages a lower surface ***523*** of the plunger ***524*** to interfere with the movement of the plunger ***524*** as previously described. As the actuator button ***516*** is depressed, the upper surface ***612*** of the extension ***598*** clears the lower surface ***523*** of the plunger .***524.*** When this occurs, the drive carrier is released to be driven by spring ***534*** for placing the cannula ***130*** into a deployed position beneath the patient's skin.

**FIG. 25** further shows that the latch ***566*** includes a second catch ***573.*** The second catch ***573*** serves to catch the hook after it is lifted over the first catch ***772.*** The second catch ***573*** is also positioned so that even though it catches the hook ***570,*** the projections are still separated sufficiently to release the cannula placement assembly ***500*** from the device ***110.*** The cannula placement assembly will not fall apart because it is held together in part by the second catch ***572*** catching the hook ***570.***

**FIG. 26** is a sectional view showing the cannula placement assembly ***500*** after cannula deployment. The assembly is separated from the device. The projection ***560*** is fully within the slot ***550*** of the control button ***518.*** This serves to further preclude reuse of the assembly ***500*** after cannula placement.

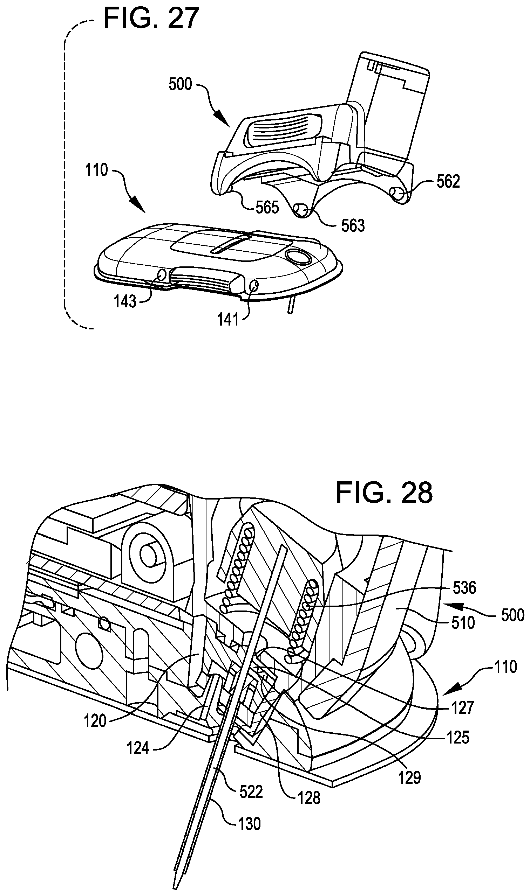

**FIG. 27** is a perspective view of the device ***110*** and cannula placement assembly ***500*** after cannula deployment and separation of the cannula placement assembly from the device. Clearly seen in **FIG. 27** are the remaining projections ***563*** and ***565*** and remaining pockets ***141*** and ***143*** for releasably retaining the assembly ***500*** on the device ***110.*** After cannula placement, the separated cannula placement assembly ***500*** may be discarded.

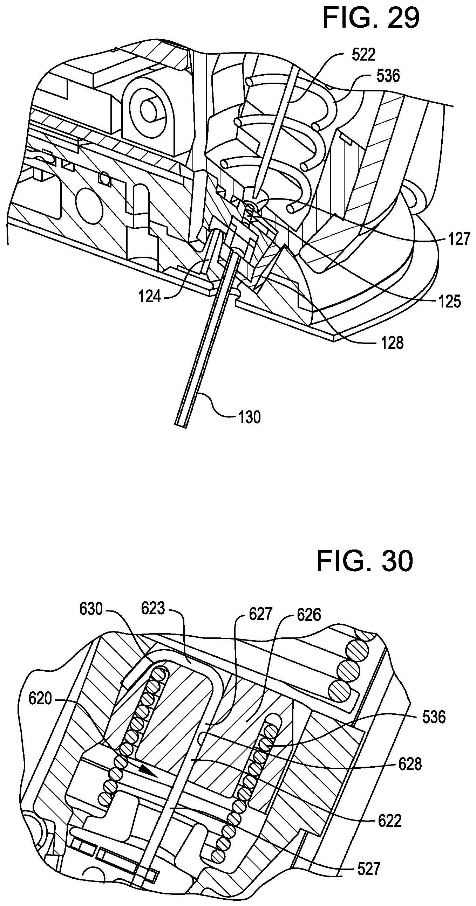

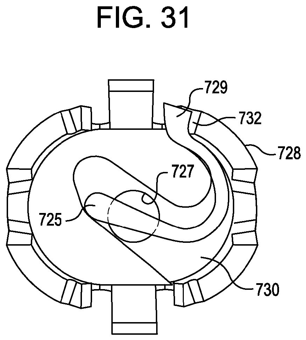

**FIG. 28** is a sectional view of the cannula placement assembly ***500*** and the device ***110*** to an enlarged scale showing details of a cannula needle port cover ***125*** during delivery of the cannula ***130.*** The cannula ***130*** is carried on the cannula carrier ***128.*** The cannula carrier includes a port ***127*** through which the needle ***522*** passes when it is retracted back into the driver ***510*** by the spring ***536.*** To preclude access into the device and especially direct access to the cannula through the port ***127*** after the cannula ***130*** has been deployed, the cannula carrier ***128*** includes the port cover ***125.*** In accordance with this embodiment, the port cover is made of relatively impenetrable material such as hard plastic or steel or the like and is substantially U-shaped and is confined within a slot ***129.*** One leg of the port cover ***125*** is displaced and held away from the port ***127*** by the needle ***522.*** It is a resilient material, such as spring steel and is held in place in, for example, a slightly compressed state, by the needle shaft ***527*** which is loaded through the hole in the port cover as shown at ***125*** in **FIG. 28****.** Responsive to the cannula ***130*** being placed and the needle shaft ***527*** removed from the device ***110,*** the one leg of the port cover is freed and permitted to spring to its natural shape such that the one leg overlies the port **127** as shown at ***125*** in **FIG. 29** and in greater detail at ***727*** in **FIG. 31****.** This results in the port ***127*** being blocked to preclude access to the interior of the device ***110*** once the cannula has been placed. Once the cannula ***130*** is deployed, the device ***110*** will appear as shown in **FIG. 2****.** As previously mentioned, when the cannula ***130*** is placed, a fluid connection is established between the outlet ***124*** and the cannula ***130*** through the cannula carrier ***128.*** After priming, the device ***110*** is ready to deliver its first dose of medicament.

**FIG 30** is a sectional view, to an enlarged scale, showing another driver needle assembly ***620*** including a drive needle shaft ***622*** and drive needle head ***626*** according to a further embodiment of the invention. Here it may be seen that the drive needle shaft ***622*** and the drive needle head ***626*** are two separate parts. The drive needle shaft ***622*** has a turned section ***623*** and an elongated section ***627.*** The turned section ***623*** rides in a slot ***630*** of the drive needle head ***626.*** The elongated section ***627*** passes through and is free to slide on a through bore ***628*** of the drive needle head ***626.*** The needle assembly ***620*** may be used to assist in cannula placement and returned by spring ***536*** after cannula deployment in the same manner as previously described.

**FIG. 31** is a sectional view, to an enlarged scale, illustrating an alternative embodiment of a cannula port cover ***725.*** The port cover is shown covering a needle port ***727*** after cannula deployment. The port ***727,*** as in the precious embodiment, is defined by the cannula carrier ***728.*** The port cover ***725*** is generally U-shaped and confined in a cut-out ***730.*** The cut-out ***730*** has a width that is greater than the diameter of the needle slot ***727*** to permit a cannula driver needle (not shown) to pass there through upon return after cannula placement. The port cover ***725*** has an end ***729*** that is confined by the cut-out ***730*** and a slot ***732*** formed in a side wall of the cannula carrier ***728.*** The port cover ***725*** is therefore free to pivot to the position shown after the cannula driver needle (not shown) passes there through upon its return.

As may be noted in **FIG. 31****,** the port cover ***725*** has a width, adjacent the port ***727,*** that is smaller than the diameter of the port ***727.*** While the port cover ***725*** does not completely cover the port ***727,*** it is still of sufficient dimension to block the port ***727*** and preclude access to the cannula through the port ***727*** by, for example, an external syringe needle.

While particular embodiments of the present invention have been shown and described, modifications may be made within the scope of the invention, which is defined by the claims. It is therefore intended in the appended claims to cover all such changes and modifications which fall within the scope of the invention as defined by those claims.

## Claims

1. A cannula assembly (122) for use within a disposable infusion device (110), comprising:
a cannula carrier (128) configured to be received within a body cavity (120) of the disposable infusion device (110);
a cannula (130) extending from the cannula carrier (128) for being beneath the patient's skin, wherein the cannula carrier (128) includes a port (127) aligned with the cannula (130) that facilitates deployment of the cannula assembly (122) within the disposable infusion device (110); and
a port cover (125) that blocks the port (127),
**characterized in that** the port cover is configured to preclude direct access by an external syringe needle to the cannula (130) through the port (127) after deployment.

2. The cannula assembly (122) of claim 1, wherein the cannula assembly is arranged to be deployed with a drive needle (522) that extends through the port (127) and carries the cannula carrier (128) and the cannula (130) into the deployed position within the infusion device, and wherein the port cover is arranged to block the port (127) upon withdrawal of the drive needle (522) from the cannula carrier (128) after deployment of the cannula assembly (122).

3. The cannula assembly (122) of claim 2, wherein the port cover (125) is arranged to be displaced from the port (127) by the drive needle (522) prior to cannula deployment.

4. The assembly of claim 3, wherein the port cover (125) is formed of resilient material and is arranged to spring over and block the port (127) responsive to the drive needle (522) being withdrawn from the port (127).

5. A disposable wearable infusion device (110) comprising:
a body arranged to be adhered to a patient's skin, the body having a cavity (120);
the cannula assembly (122) of any one of claims 1 to 4;
a reservoir (180) for holding a liquid medicament to be infused into the patient; and
a pump (172) that displaces a volume of the liquid medicament from the reservoir (180) to the cannula (130) for delivery to the patient.

6. The device of claim 5, wherein the cannula carrier (128) includes the port cover (125).

## Patentansprüche

1. Kanülenanordnung (122) zur Verwendung innerhalb einer Einweg-Infusionsvorrichtung (110), umfassend:
einen Kanülenträger (128), der konfiguriert ist, um innerhalb einer Körperhöhle (120) der Einweg-Infusionsvorrichtung (110) aufgenommen zu werden;
eine Kanüle (130), die sich von dem Kanülenträger (128) erstreckt, um unter der Haut des Patienten zu liegen, wobei der Kanülenträger (128) einen Anschluss (127) einschließt, der mit der Kanüle (130) ausgerichtet ist, der einen Einsatz der Kanülenanordnung (122) innerhalb der Einweg-Infusionsvorrichtung (110) erleichtert; und
eine Anschlussabdeckung (125), die den Anschluss (127) blockiert,
**dadurch gekennzeichnet, dass** die Anschlussabdeckung konfiguriert ist, um einen direkten Zugriff durch eine externe Spritzennadel auf die Kanüle (130) durch den Anschluss (127) nach dem Einsatz zu verhindern.

2. Kanülenanordnung (122) nach Anspruch 1, wobei die Kanülenanordnung eingerichtet ist, um mit einer Antriebsnadel (522) eingesetzt zu werden, die sich durch den Anschluss (127) erstreckt und den Kanülenträger (128) und die Kanüle (130) in die eingesetzte Position innerhalb der Infusionsvorrichtung trägt, und wobei die Anschlussabdeckung eingerichtet ist, um den Anschluss (127) bei einem Herausziehen der Antriebsnadel (522) aus dem Kanülenträger (128) nach dem Einsatz der Kanülenanordnung (122) zu blockieren.

3. Kanülenanordnung (122) nach Anspruch 2, wobei die Anschlussabdeckung (125) eingerichtet ist, um von dem Anschluss (127) durch die Antriebsnadel (522) vor dem Einsatz der Kanüle verdrängt zu werden.

4. Anordnung nach Anspruch 3, wobei die Anschlussabdeckung (125) aus elastischem Material besteht und eingerichtet ist, um über den Anschluss (127) zu springen und diesen zu blockieren, als Reaktion darauf, dass die Antriebsnadel (522) aus dem Anschluss (127) herausgezogen wird.

5. Tragbare Einweg-Infusionsvorrichtung (110), umfassend:
einen Körper, der eingerichtet ist, um an der Haut eines Patienten angehaftet zu werden, wobei der Körper einen Hohlraum (120) aufweist;
die Kanülenanordnung (122) nach einem der Ansprüche 1 bis 4;
einen Behälter (180) zum Lagern eines flüssigen Medikaments, das dem Patienten infundiert werden soll; und
eine Pumpe (172), die ein Volumen des flüssigen Medikaments aus dem Behälter (180) in die Kanüle (130) verdrängt, um es dem Patienten zuzuführen.

6. Vorrichtung nach Anspruch 5, wobei der Kanülenträger (128) die Anschlussabdeckung (125) einschließt.

## Revendications

1. Ensemble canule (122) pour utilisation au sein d'un dispositif de perfusion jetable (110), comprenant :
un support de canule (128) conçu pour être reçu au sein d'une cavité de corps (120) du dispositif de perfusion jetable (110) ;
une canule (130) s'étendant à partir du support de canule (128) pour être en dessous de la peau du patient, dans lequel le support de canule (128) comporte un orifice (127) aligné avec la canule (130) qui facilite le déploiement de l'ensemble canule (122) au sein du dispositif de perfusion jetable (110) ; et
un couvercle d'orifice (125) qui bloque l'orifice (127),
**caractérisé en ce que** le couvercle d'orifice est conçu pour empêcher un accès direct, par une aiguille de seringue externe, à la canule (130) à travers l'orifice (127) après déploiement.

2. Ensemble canule (122) selon la revendication 1, dans lequel l'ensemble canule est agencé pour être déployé avec une aiguille d'entraînement (522) qui s'étend à travers l'orifice (127) et transporte le support de canule (128) et la canule (130) dans la position déployée au sein du dispositif de perfusion, et dans lequel le couvercle d'orifice est agencé pour bloquer l'orifice (127) lors du retrait de l'aiguille d'entraînement (522) du support de canule (128) après déploiement de l'ensemble canule (122).

3. Ensemble canule (122) selon la revendication 2, dans lequel le couvercle d'orifice (125) est agencé pour être déplacé de l'orifice (127) par l'aiguille d'entraînement (522) avant un déploiement de canule.

4. Ensemble selon la revendication 3, dans lequel le couvercle d'orifice (125) est formé de matériau élastique et est agencé pour se replier et bloquer l'orifice (127) en réponse à l'aiguille d'entraînement (522) étant retirée de l'orifice (127).

5. Dispositif de perfusion jetable (110) pouvant être porté, comprenant :
un corps agencé pour être fixé par adhérence à la peau d'un patient, le corps ayant une cavité (120) ;
l'ensemble canule (122) selon l'une quelconque des revendications 1 à 4 ;
un réservoir (180) permettant de retenir un médicament liquide à perfuser dans le patient ;
et
une pompe (172) qui déplace un volume du médicament liquide à partir du réservoir (180) vers la canule (130) pour administration au patient.

6. Dispositif selon la revendication 5, dans lequel le support de canule (128) comporte le couvercle d'orifice (125).
